# EUROPEAN PATENT APPLICATION

(11) **EP 4 807 659 A1**
(43) Date of publication of application: **16.09.2026**
(21) Application number: 23962269.9
(22) Date of filing: 22.12.2023
(51) Int. Cl.: G06Q 50/10

(54) **VIDEO OUTPUT SYSTEM, VIDEO OUTPUT METHOD, AND PROGRAM**

(71) Applicant: Curves Japan Co., Ltd., Minato-ku Tokyo 108-0023 (JP)
(72) Inventor: MASUMOTO, Takeshi, Tokyo 108-0023 (JP); HAMAMIYA, Keiko, Tokyo 108-0023 (JP); HIGASHIYAMAZAKI, Yukino, Tokyo 108-0023 (JP); KOGA, Naotaro, Tokyo 108-0023 (JP)
(74) Representative: Hasegawa, Kan
(86) International application number: PCT/JP2023/046103
(87) International publication number: WO 2025/134349

(57) **Abstract**

**[Problem]**

To provide a novel video output system, video output method, and program that enable each user to obtain an appropriate exercise effect.

**[Solution]**

A video output system according to the present disclosure comprises: a storage unit that stores a plurality of video data items serving as exercise models; and a control unit that causes video data at an exercise level selected for each user from among the plurality of video data items and a captured image of the user to be output to a display unit provided for each user, wherein the control unit outputs, to display units arranged in a plurality of individual areas respectively used by users, the video data synchronized with each other based on predetermined order information.

## Description

### [Technical Field]

The present disclosure relates to a video output system, a video output method, and a program.

### [Background Art]

Conventionally, various exercises for the purpose of maintaining health, restoring physical condition, and the like, such as body stretching, training, dieting, rehabilitation, and the like, have been performed regardless of sex or age. The effects of these exercises can be further improved by performing the exercises while following guidance by an instructor, a video guide, or the like.

For example, Patent Document 1 discloses a system that enables a user to exercise while watching and listening to pre-recorded video and audio serving as an exercise model.

### [Prior Art Documents]

### [Patent Documents]

[Patent Document 1] Japanese Patent Application Publication No. 2023-6961

### [Summary of the Invention]

### [Problems to be Solved by the Invention]

According to the above system, the effect of exercise can be enhanced. However, there is room for further improvement in terms of enabling each user to obtain an appropriate exercise effect.

The present disclosure has therefore been made in view of the above problems, and an object thereof is to provide a novel video output system, video output method, and program that enable each user to obtain an appropriate exercise effect.

### [Means for Solving the Problems]

According to the present disclosure, there is provided a video output system comprising:
a storage unit that stores a plurality of video data items serving as exercise models; and
a control unit that causes video data at an exercise level selected for each user from among the plurality of video data items and a captured image of the user to be output to a display unit provided for each user,
wherein the control unit outputs, to display units arranged in a plurality of individual areas respectively used by users, the video data synchronized with each other based on predetermined order information.

According to the present disclosure, there is also provided a video output method in which an information processing apparatus executes:
a storage process of storing a plurality of video data items serving as exercise models; and
a control process of causing video data at an exercise level selected for each user from among the plurality of video data items and a captured image of the user to be output to a display unit provided for each user,
wherein the control process includes a process of outputting, to display units arranged in a plurality of individual areas respectively used by users, the video data synchronized with each other based on predetermined order information.

According to the present disclosure, there is also provided a program causing an information processing apparatus to execute:
a storage process of storing a plurality of video data items serving as exercise models; and
a control process of causing video data at an exercise level selected for each user from among the plurality of video data items and a captured image of the user to be output to a display unit provided for each user,
wherein the control process includes a process of outputting, to display units arranged in a plurality of individual areas respectively used by users, the video data synchronized with each other based on predetermined order information.

### [Effects of the Invention]

According to the present disclosure, it is possible to provide a novel video output system, video output method, and program that enable each user to obtain an appropriate exercise effect.

### [Brief Description of the Drawings]

[FIG. 1] FIG. 1 is a diagram illustrating a configuration example of a system according to an embodiment of the present disclosure.
[FIG. 2] FIG. 2 is a diagram illustrating a configuration example of an information processing apparatus according to the embodiment.
[FIG. 3] FIG. 3 is a diagram illustrating an example of user information according to the embodiment.
[FIG. 4] FIG. 4 is a diagram illustrating another example of user information according to the embodiment.
[FIG. 5] FIG. 5 is a diagram illustrating a display example of video in the system according to the embodiment.
[FIG. 6] FIG. 6 is a diagram illustrating an example of an exercise menu according to the embodiment.
[FIG. 7] FIG. 7 is a diagram illustrating an example of an exercise menu applied to a user according to the embodiment.
[FIG. 8] FIG. 8 is a flowchart relating to a series of control operations in the system according to the embodiment.
[FIG. 9] FIG. 9 is a diagram illustrating an example of a screen of a display unit according to the embodiment.
[FIG. 10] FIG. 10 is a diagram illustrating another example of the screen of the display unit according to the embodiment.
[FIG. 11] FIG. 11 is a diagram illustrating another example of the screen of the display unit according to the embodiment.
[FIG. 12] FIG. 12 is a diagram illustrating another example of the screen of the display unit according to the embodiment.

### [Description of Embodiments]

A preferred embodiment of the present disclosure will be described in detail below with reference to the accompanying drawings. In the present specification and drawings, components having substantially the same functional configurations are denoted by the same reference signs, and redundant description thereof is omitted.

The system of the present embodiment provides video suitable for various exercises for purposes such as maintaining or improving health, restoring physical condition, improving pain, and the like, including body stretching, training, dieting, rehabilitation, and the like. A user can enhance an exercise effect by moving the body so as to imitate a model video while comparing a video serving as a model for movement displayed on a display unit with a video obtained by capturing the user himself or herself.

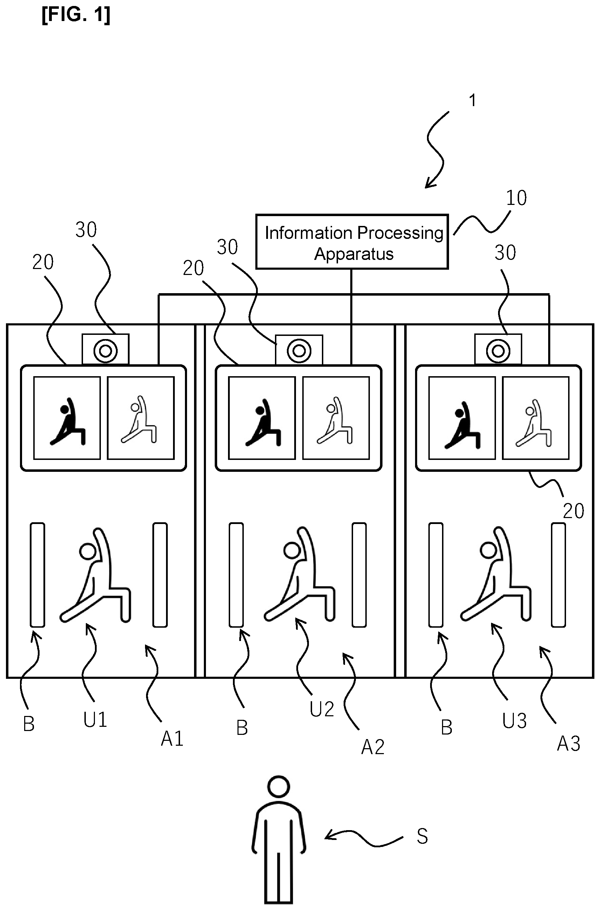

FIG. 1 is a schematic diagram illustrating an example of a video output system 1 of the present embodiment. The present system includes an information processing apparatus 10 for providing video to users, a display unit 20 configured by a monitor or the like, and a camera 30. The information processing apparatus 10, the display unit 20, and the camera 30 are connected to each other by wires, but may transmit and receive various types of information by communicating with each other through wireless connection.

In the example of FIG. 1, a plurality of individual areas for exercise (so-called individual booths) A1, A2, A3, ... are partitioned and provided in an indoor space such as a store or a home used by a plurality of users, and each of users U1, U2, U3, ... can exercise while looking at a monitor (20) provided in a corresponding individual area. A support member B such as a handrail for supporting the exercise of the user is provided in each individual area. The support member B can be used, by placing or hooking a hand or foot thereon, to enhance an exercise effect or prevent a fall during exercise. In addition, tools or equipment that assist exercise, such as a chair, a bed, a step, a ball, a roller, and the like, may be arranged in the individual area. In the example of FIG. 1, one staff member S is illustrated, and is configured to be able to check the exercise states of the plurality of users and give advice or support. The staff member S is not essential, and is not limited to one person; a plurality of staff members may be provided. The present system is not limited to the illustrated example, and may be configured, for example, such that only one display unit is provided in one individual area and a user exercises alone.

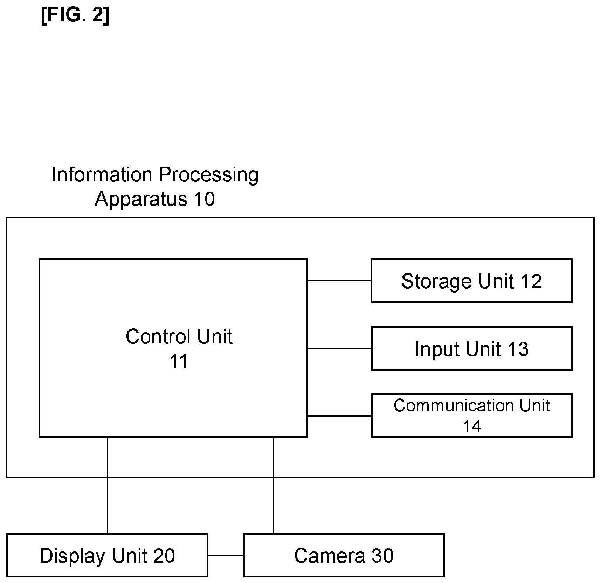

FIG. 2 is a block diagram illustrating a functional configuration of the information processing apparatus 10. The information processing apparatus 10 is an apparatus used by a system administrator or the like when operating and managing various services, and may be, for example, a general-purpose computer such as a workstation or a personal computer, or may be logically realized by cloud computing technology. The information processing apparatus 10 is not limited to a single apparatus, and may be configured by combining a plurality of apparatuses, or may be configured by a server apparatus disposed at another location and information processing terminals at respective stores.

The information processing apparatus 10 may include, for example, as illustrated in FIG. 2, a control unit 11, a storage unit 12, an input unit 13, and a communication unit 14. The information processing apparatus 10 can receive various types of input information input via the input unit 13, the communication unit 14, the camera 30, and the like. The information processing apparatus 10 can execute processing by a program corresponding to the input information in the control unit 11, and output a processing result of the program. The output information may include, for example, video (moving images), images, audio, and the like, and may be output from the display unit 20 or another output unit, or may be transmitted to an external apparatus or the like via the communication unit 14. A part of the program may be transmitted to another information processing apparatus and executed on the other information processing apparatus. In this case, the other information processing apparatus may be, for example, a smartphone, a mobile phone terminal, a tablet terminal, a personal computer, or the like used by a staff member or a user, and may be connected to the information processing apparatus 10 wirelessly or by wire via a network such as the Internet.

The control unit 11 transfers information between respective units and controls the entire information processing apparatus 10, and is realized, for example, by a CPU (Central Processing Unit), an MPU (Micro Processing Unit), or a GPU (Graphics Processing Unit) executing a program stored in a predetermined memory.

The storage unit 12 stores various types of information. The storage unit 12 can store programs for executing various control processes and respective functions in the control unit 11, input information, and the like, and is configured by one of, or any combination of, RAM (Random Access Memory), ROM (Read Only Memory), flash memory, an HDD (Hard Disk Drive), an SSD (Solid State Drive), other storage, and the like.

The storage unit 12 can store user information regarding users, exercise menu information regarding exercise menus, and the like.

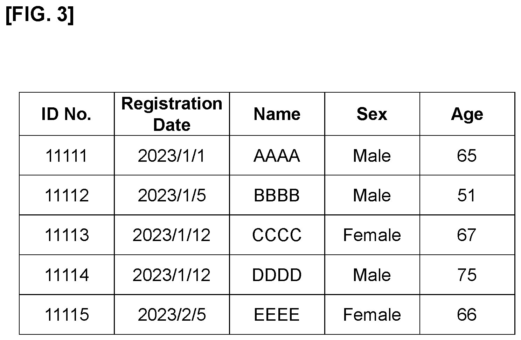

As illustrated, for example, in FIG. 3, the user information may include information such as identification information unique to each user (user ID), registration date, name, and the like, and user attribute information such as sex and age (including age-group information such as teens, twenties, and the like).

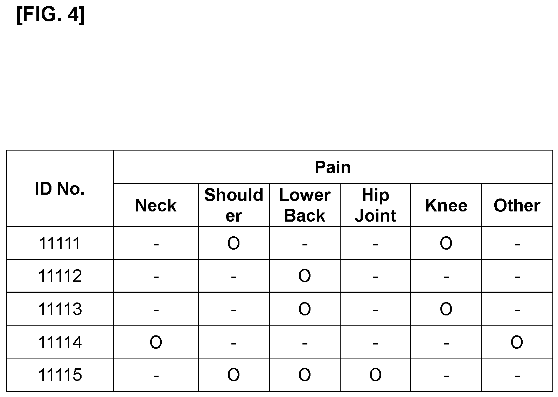

As illustrated, for example, in FIG. 4, the user information may include information regarding painful body parts of each user. The information regarding painful body parts is stored in the storage unit, for example, by the user himself or herself or the staff member S inputting the information via the input unit 13 before exercise. In the example of FIG. 4, items including neck, shoulders, lower back, hip joints, knees, and others are included; a painful part is indicated by "O," and a part without pain is indicated by "-." For example, the control unit 11 can display, on the display unit 20 or on a display device such as a smartphone or a tablet terminal used by a user or a staff member, the items of neck, shoulders, lower back, hip joints, knees, and others in a selectable input state, receive input information regarding a painful part selected by the user or staff member, and store the input information in the storage unit. The user information may also include information such as a history of exercises in which each user used the present system, a history of painful body parts, and the like. For example, the exercise history may include date/time information such as the date, time, and the like when a user performed exercise using the present system, the type, difficulty, order (start menu, end menu), and the like of an exercise menu performed (output video data), and the pain history may be information in which date/time information at which a painful part was input is associated with the painful part.

The input unit 13 is used by a user, a staff member, a system administrator, or the like to input various types of information, and is realized by, for example, a keyboard, a mouse, a touch panel, a microphone, or the like. The user or the staff member can input various types of information regarding the user, select an exercise menu, and perform input operations such as starting, stopping, temporarily stopping, and resuming exercise via the input unit 13.

The communication unit 14 is used to communicate with another information processing apparatus, and has a function as a receiving unit that receives various data and signals transmitted from another information processing apparatus or the like, and a function as a transmitting unit that transmits various data and signals to another information processing apparatus or the like in response to an instruction from the control unit 11.

The display unit 20 can output, as images, information generated by the control unit 11 and information received via the communication unit. The display unit 20 is configured by, for example, a monitor such as a liquid crystal display (LCD), a touch panel, or the like. In addition to the display unit 20, an audio output unit such as a speaker may be provided at an arbitrary position in an individual area (A1, A2, A3), or audio may be output from headphones or earphones used by each user. An output unit such as the display unit 20 can output various types of information based on an instruction from the control unit 11. The information processing apparatus 10 may include an output unit for outputting images (moving images) and audio.

The camera 30 can capture a user located in an individual area, and can display image data on the display unit 20 in real time or transmit the image data to the information processing apparatus 10. In the illustrated example, the camera 30 is provided above the display unit 20, but is not limited thereto, and may be provided below the display unit 20, on either the left or right side, integrated with the display unit 20, or provided at a position separated from the display unit 20. A plurality of cameras 30 may be provided for each individual area. For example, two, three, or more cameras may be provided, such as one camera capable of capturing the front of a user, one camera capable of capturing a side of the user, and one camera capable of capturing the back of the user.

In an individual area, a camera for image recognition of an identification code such as a barcode or a two-dimensional code associated with identification information of a user, or an apparatus such as a tablet terminal including the camera, may be installed. For example, by reading, with the camera, a barcode printed on a membership card or the like used by each user, information regarding the user associated with the barcode and prestored in the storage unit may be acquired. According to this, reception processing can be performed efficiently using the barcode, so that the time until the start of exercise can be shortened. Image data acquired by the camera can be subjected to various image analyses by the control unit of the information processing apparatus, but the camera itself may be provided with the function of the control unit. The camera 30 illustrated in FIG. 1 may be used to recognize a barcode or the like.

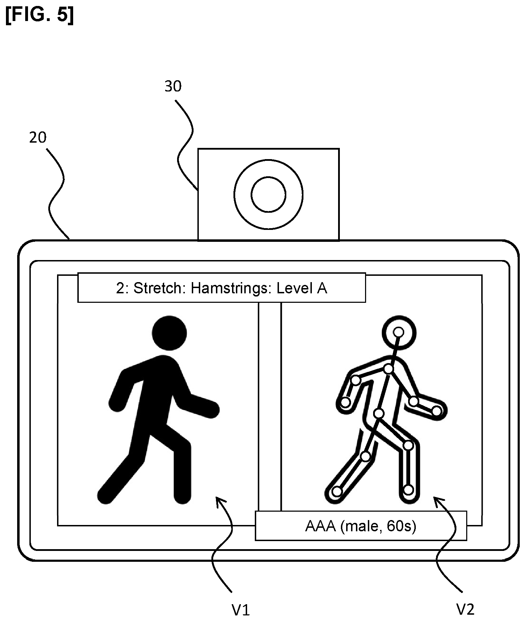

As illustrated in FIG. 5, the control unit 11 can cause the display unit 20 to display a video V1 (video data) serving as an exercise model and a real-time video V2 of the user himself or herself captured by the camera 30. The control unit 11 may superimpose skeletal information of the user on the video V2 by performing image analysis on captured data of the camera 30. For example, a plurality of circle marks indicating positions of predetermined joints and the like of the user (ankles, knees, waist, shoulders, elbows, wrists, head, and the like), and lines connecting the circle marks, may be shown. According to this, the user can exercise while checking his or her skeleton and posture, and the exercise effect can be enhanced. The model video V1 and the user's captured image V2 are not limited to the arrangement in FIG. 5, and may be displayed side by side with the left and right positions reversed, arranged vertically, or superimposed while making one transparent. As illustrated in FIG. 5, the control unit 11 may display information regarding the displayed exercise menu (number, type, target part, level), and may display user information (name, sex, age, painful part, and the like).

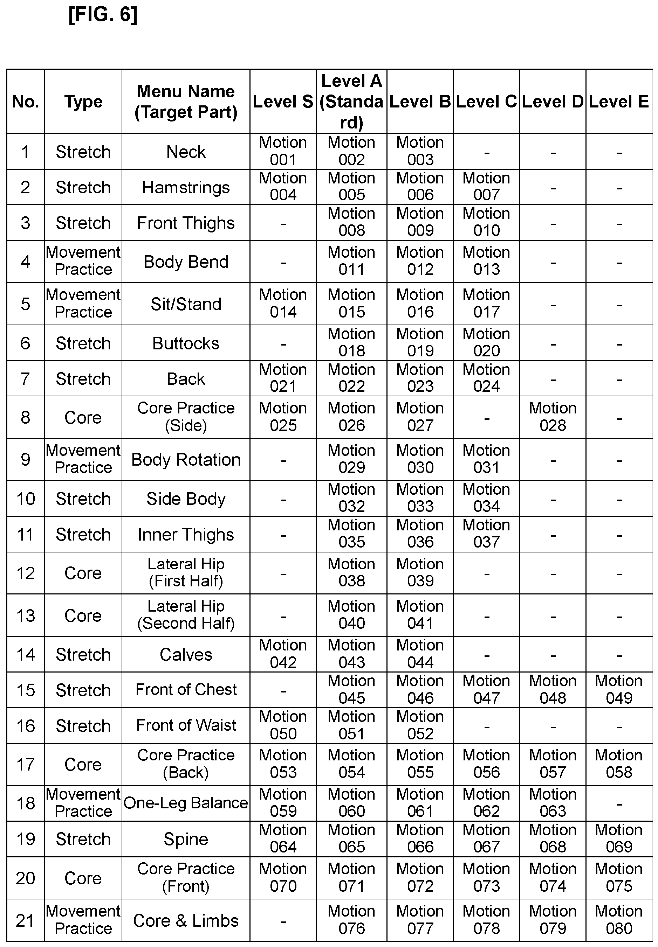

Specific contents of the video V1 serving as the exercise model will now be described. FIG. 6 illustrates an example of exercise menu information displayed as the video V1. The exercise menu proceeds according to order information from number 1 to number 2, number 3, ... number 21, that is, from smaller numbers to larger numbers, and after number 21 ends, the exercise menu returns to number 1 and is repeatedly displayed on the display unit as number 2, number 3, and so on. Each user may start exercise from number 1 of the exercise menu, or may start from an exercise menu number that is not number 1, such as from number 6 or number 10. Regardless of the number from which the exercise menu starts, the exercise basically ends when the exercise menu has completed one cycle (the 21 menus once); however, exercise may be performed over a plurality of cycles, such as two or three cycles, for a predetermined number of times or a number of times selected by the user or a staff member.

In the example of FIG. 6, the exercise menu information includes video data associated with information such as number (order information), type (attribute information), menu name, and level (exercise level). In the present example, the numbers are numbers 1 to 21, but are not limited thereto, and may be fewer or greater than 21. The exercise menu information can be arbitrarily set by a service provider (system administrator) and prestored in the storage unit.

The types of exercise menus are preliminarily classified into three types: stretching, core, and movement practice. The types are not limited to three, and may be two or fewer, or four or more. In the present example, "stretching" is exercise for improving muscle flexibility, "core" is exercise for improving core stability, and "movement practice" is exercise for improving coordination between the brain and the body.

The menu name is a name for each menu that represents the purpose of the exercise or a body part to be loaded, and an arbitrary name can be set by the service provider.

A plurality of stages are set for each exercise menu level. The levels are set such that level S has the greatest exercise load and the highest degree of difficulty, and the load gradually decreases and the degree of difficulty becomes lower in the order of level A, level B, ... level E. As an example of the number of levels, the exercise menu of number 12 "lateral hip (first half)" in FIG. 6 is set in two stages, level A and level B. In the example of FIG. 6, the maximum number of stages is six and the minimum number is two, but only one stage may be set, or seven or more stages may be set. A level can be selected based on user information such as the exercise ability, pain, age, sex, and the like of the user. The exercise menu may be selected by the staff member S or the user himself or herself, or may be automatically selected by the control unit based on a predetermined program. In the present example, a first-time user having no input of a painful part is configured such that level A is selected as a standard exercise level, but the configuration is not limited thereto.

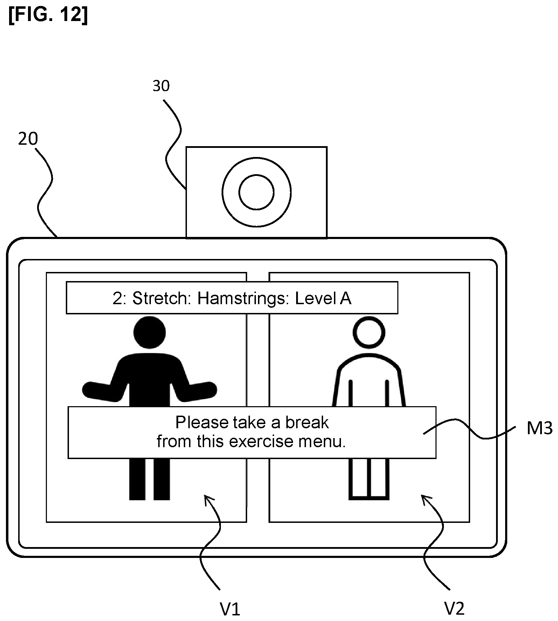

As exercise menu information, video data (moving image data) of exercise menus associated with individual exercise menu identification information from "Motion 001" to "Motion 080" is stored in the storage unit. The moving images from "Motion 001" to "Motion 080" are prestored moving images that have been captured, edited, and the like in advance, and all of them may be moving images having the same length of time. The lengths of the moving images may be different from each other, but it is preferable that at least moving images having the same menu number (for example, "Motion 001," "Motion 002," and "Motion 003," which have number 1) have the same length of time. For each of the numbers 1 to 21 (numbers indicating order) in the exercise menu, one exercise level from level S to level E is selected for each user, and the corresponding model video of "Motion XXX" is displayed on the display unit 20 as the model video V1. When there is no corresponding video data (corresponding to "-" in FIG. 6), the video data is not displayed and, for example, a message indicating that the exercise of that number is to be rested can be displayed. For example, in the exercise menu "neck" of number 1, the menu having the lowest load level is "Motion 003" of level B. In this case, for a user having neck pain or a user having weak neck muscle strength, the video of the minimum level "Motion 003" may be displayed; alternatively, the model video may not be displayed and a message such as "Please take a break from this exercise menu" may be displayed to prompt the user to wait without performing a specific exercise menu (see FIG. 12). That is, in the present system, based on the user information, the model videos of all numbers of the exercise menu may be displayed, the model video of a specific numbered exercise menu may not be displayed, or a display prompting the user not to exercise may be provided.

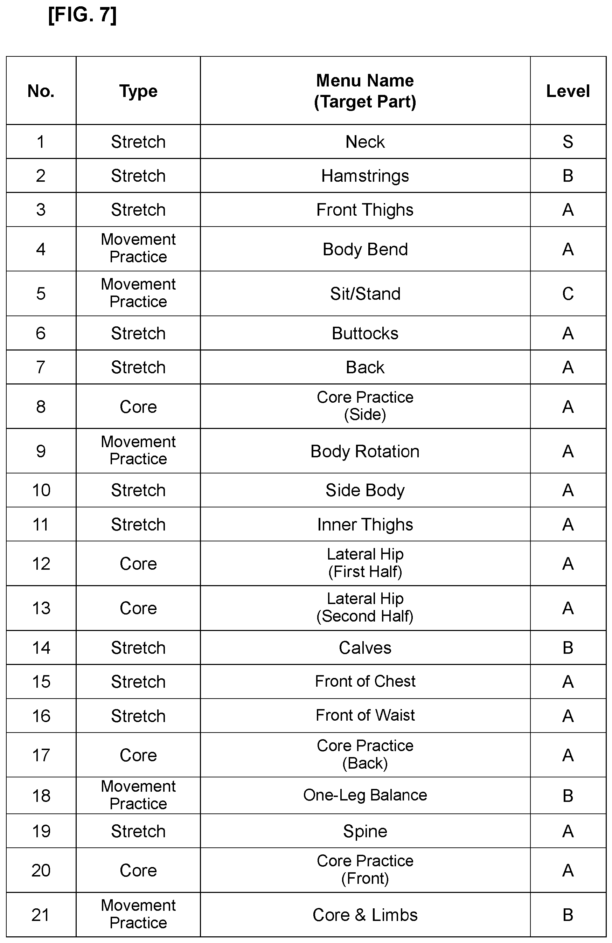

FIG. 7 illustrates an example of exercise levels applied to respective users. As illustrated in FIG. 7, one level is selected for each of menu numbers 1 to 21 for each user, and model video data corresponding to the selected level is displayed.

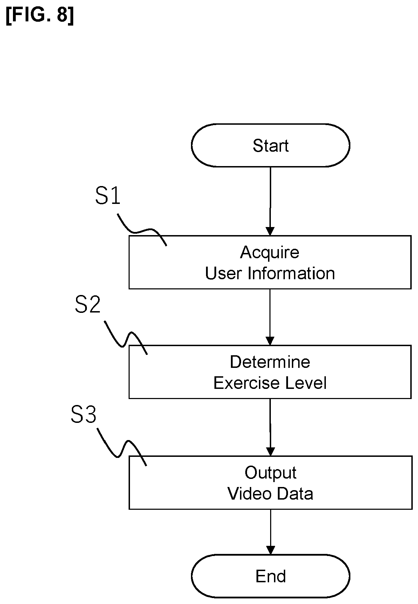

FIG. 8 illustrates a flow of information processing in the present system. First, a user inputs user information via the input unit 13 through the staff member S or by himself or herself. The staff member S can ask the user for information as needed and input the user information. As a result, the control unit of the information processing apparatus acquires the user information (S1).

Specifically, a user using the present system for the first time can register user information by inputting information such as his or her name, sex, age, painful parts, and the like, either by himself or herself or through a staff member. When receiving the input information, the control unit can assign an identification number and barcode information unique to the user, acquire date/time information at that point in time, and store the date/time information in the storage unit in association with the input information (see FIGS. 3 and 4). The service provider can provide each user with a membership card or the like printed with a registered barcode unique to that user. Thus, a user using the present system for the second time or thereafter can read information in the storage unit without inputting user information every time, by causing the camera of the individual area to recognize the barcode of the membership card. Certain user information such as pain information may be input every time so as to update the information in the storage unit.

The control unit of the information processing apparatus determines exercise levels in the exercise menus 1 to 21 based on the user information (S2). The control unit can determine the exercise menu based on at least one of the user's age, sex, painful part, and past history information, or a combination of a plurality of pieces of information. The exercise menu may be determined automatically by the control unit based on predetermined conditions, or may be determined by a staff member based on the user information. For example, for an exercise menu of a target part having pain, a lower level can be selected than when there is no pain. Further, a higher level can be selected for the exercise menu as a whole as the age is younger. Further, a higher level as a whole may be selected for men than for women. As illustrated in FIG. 7, the levels of all exercise menus are determined for each user. Returning to FIG. 8, the control unit outputs video data corresponding to the level to the display unit (S3). At the same time, the control unit operates the camera 30 and outputs a captured image of the user in real time to the display unit.

In the present example, the video V1 displayed as a model is synchronized in all individual areas A1, A2, A3, ... . That is, video data of the same menu number is displayed at the same timing in all individual areas A1, A2, A3, ... . For example, while the video menu of level A of number 3 (Motion 008) is displayed in the individual area A1 used by the user U1, a video menu of any level of number 3 (any of Motion 008, Motion 009, and Motion 010) is displayed in the individual areas A2 and A3 according to the user information. By displaying synchronized exercise menus in a plurality of individual areas in this way, a plurality of users can enjoy the fun of exercising together, and motivation for exercise can be enhanced. A video menu at a different level may be displayed for each user even for the same number. Even when the levels differ, exercise menus having the same number have a common target part, and therefore an effect can be obtained in which a plurality of users perform similar exercise together.

FIG. 8 illustrates an example of a flow for displaying video using the present system. Users using the present system can each visit a store or the like and exercise at an arbitrary timing.

Here, in the present system, regardless of the presence or absence of a user, the control unit may repeatedly display model videos on the display unit at a constant cycle in the order of the menu numbers. In that case, the user can start the exercise menu at an arbitrary timing. For example, when the user starts exercise from number 1, the user exercises along the exercise menu in order of number 2, number 3, ... and ends at number 21. When the user starts, for example, from number 2, the user exercises along the exercise menu in order of number 3, number 4, ... and ends at number 1.

The control unit can receive a start input based on input information of a user or the staff member S via the input unit 13. Alternatively, the start input may be received by recognizing a barcode.

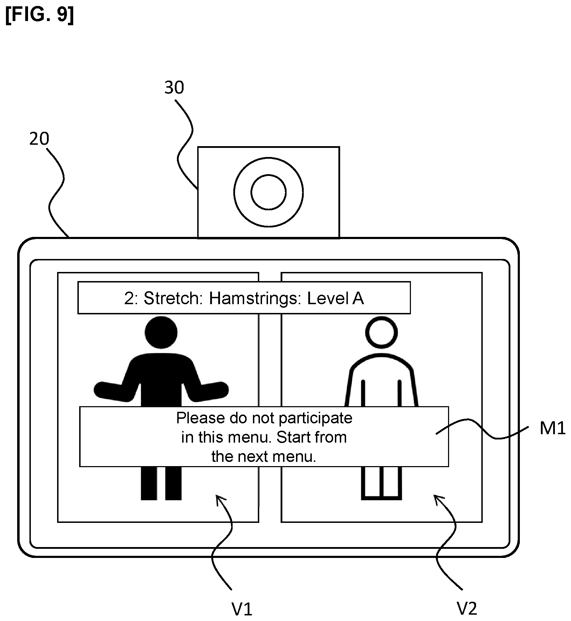

When receiving the start input, the control unit may determine a timing at which the user is to start exercise. For example, the control unit can display video so that the user performs exercise only from an exercise menu of a predetermined specific type (attribute information) or from a specific menu number. Specifically, in order to allow exercise to start from a menu corresponding to the "stretching" type, which places a small burden on the body, during a time in which "core" and "movement practice" menus are displayed, the model video and the captured image may be controlled not to be displayed, or a message such as "Please do not participate in this menu. Start from the next menu." may be displayed as illustrated in FIG. 9.

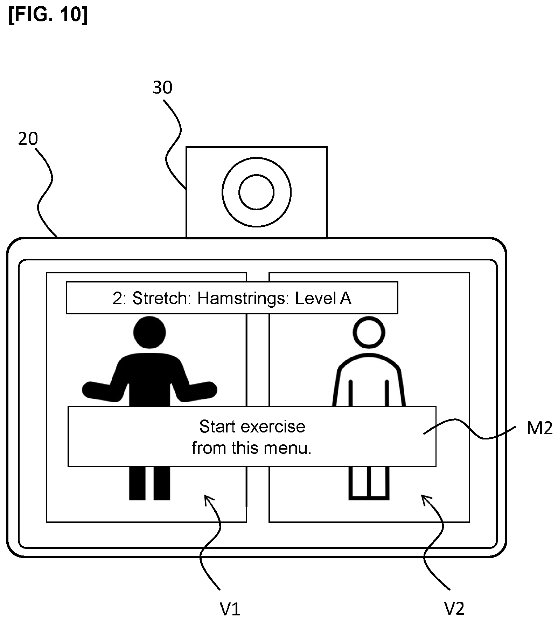

The control unit may output image data prompting the start of exercise to the display unit at a timing determined based on the start input. For example, as illustrated in FIG. 10, at a timing prompting the start of exercise, the control unit can cause the display unit to output image data of a message such as "Start exercise from this menu." This timing can be arbitrarily set by the service provider and stored in the storage unit. For example, the timing may be a menu immediately after the start input is received; among menus having a specific attribute (for example, stretching), a menu that is reproduced at the earliest timing after the start input is received; or among menus having a specific number (for example, even numbers, odd numbers, multiples of 5, and the like), a menu that is reproduced at the earliest timing after the start input is received. By prompting the start of exercise at an appropriate timing in this way, the user can easily grasp the timing at which exercise starts, and convenience can be improved. In addition, for example, by prompting the user to start from a menu having a small load, injury can be prevented.

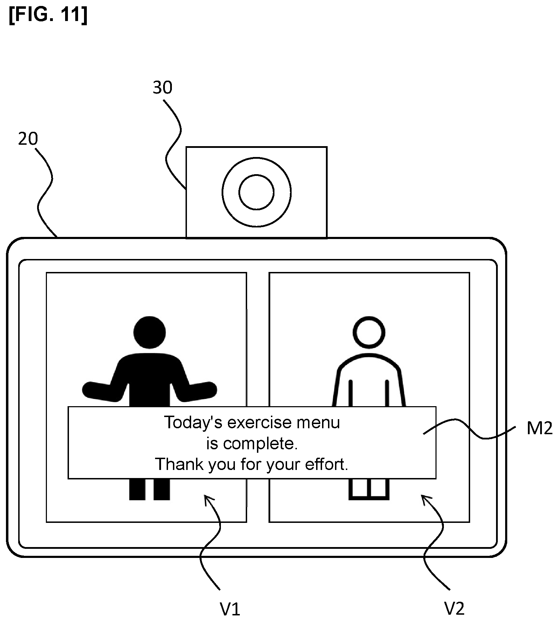

The control unit can output image data prompting the end of exercise to the display unit at a predetermined timing. This timing can be arbitrarily set by the service provider and stored in the storage unit. For example, the control unit may output the image data prompting the end of exercise to the display unit after a predetermined period from the output of the image data prompting the start of exercise. For example, at a timing when the exercise menu has completed one cycle after the image data prompting the start is output, the control unit can cause the display unit to output image data of a message such as "Today's exercise menu is complete. Thank you for your effort." as illustrated in FIG. 11. By prompting the end of exercise at an appropriate timing in this way, excessive exercise can be prevented and injury can be prevented.

The control unit may also output image data prompting the user not to perform an exercise menu based on the user information. For example, as illustrated in FIG. 12, image data prompting a temporary stop of exercise, such as "Please take a break from this exercise menu." may be output. According to this, for a user having pain in a specific part, exercise targeting that part can be avoided, and injury can be prevented.

As described above, the video output system of the present embodiment comprises: a storage unit that stores a plurality of video data items serving as exercise models; and a control unit that causes video data at an exercise level selected for each user from among the plurality of video data items and a captured image of the user to be output to a display unit provided for each user, wherein the control unit outputs, to display units arranged in a plurality of individual areas respectively used by users, the video data synchronized with each other based on predetermined order information. With such a configuration, an exercise level is selected for each user, and therefore each user can perform appropriate exercise. Accordingly, each user can obtain an appropriate exercise effect. Furthermore, by a plurality of users exercising using synchronized videos as models, the users can feel a sense of unity and can increase their motivation for exercise.

In the present embodiment, the control unit may repeatedly output the plurality of video data items to the display unit at a constant cycle based on the order information. According to this, the user can start exercise at an arbitrary timing while the exercise menu is being repeated.

In the present embodiment, the control unit may select the exercise level based on information regarding a painful body part of each user. According to this, by selecting an appropriate exercise level according to the painful body part, pain can be improved, or an increase in pain due to excessive exercise can be suppressed.

In the present embodiment, the control unit may select the exercise level based on information regarding the age of each user. According to this, exercise at an appropriate level according to age can be prompted.

In the present embodiment, the control unit may select the exercise level based on information regarding the sex of each user. According to this, exercise at an appropriate level according to differences in exercise ability due to sex can be prompted.

In the present embodiment, the storage unit may store history information regarding a history of each user using the video output system, and the control unit may select the exercise level based on the history information of each user. According to this, exercise at an appropriate level according to past exercise history can be prompted. Specifically, the same exercise level as the exercise level performed in the past may be selected, or a level higher or lower than the exercise level performed in the past may be selected. Alternatively, information on a past painful body part may be used as a current painful body part, and an exercise level corresponding to that painful body part may be selected.

In the present embodiment, the control unit may output image data prompting a user to start exercise to the display unit based on a start input from the user. According to this, the user can easily understand the timing at which exercise starts, and the convenience of the system can be improved.

In the present embodiment, the control unit may output image data prompting the end of exercise to the display unit after a predetermined period from the output of the image data prompting the start of exercise. According to this, the user can easily understand the timing at which exercise ends, and the convenience of the system can be improved.

In the present embodiment, attribute information regarding a type of exercise may be associated with each of the video data items, and the control unit may determine a timing at which the start of exercise is prompted based on the start input and the attribute information. According to this, exercise can be started from an exercise menu having an appropriate attribute, and the exercise effect can be further enhanced.

A video output method according to the present embodiment is a method in which an information processing apparatus executes: a storage process of storing a plurality of video data items serving as exercise models; and a control process of causing video data at an exercise level selected for each user from among the plurality of video data items and a captured image of the user to be output to a display unit provided for each user, wherein the control process includes a process of outputting, to display units arranged in a plurality of individual areas respectively used by users, the video data synchronized with each other based on predetermined order information. With such a configuration, an exercise level is selected for each user, and therefore each user can perform appropriate exercise. Accordingly, each user can obtain an appropriate exercise effect. Furthermore, by a plurality of users exercising using synchronized videos as models, the users can feel a sense of unity and can increase their motivation for exercise.

A program according to the present embodiment causes an information processing apparatus to execute: a storage process of storing a plurality of video data items serving as exercise models; and a control process of causing video data at an exercise level selected for each user from among the plurality of video data items and a captured image of the user to be output to a display unit provided for each user, wherein the control process includes a process of outputting, to display units arranged in a plurality of individual areas respectively used by users, the video data synchronized with each other based on predetermined order information. With such a configuration, an exercise level is selected for each user, and therefore each user can perform appropriate exercise. Accordingly, each user can obtain an appropriate exercise effect. Furthermore, by a plurality of users exercising using synchronized videos as models, the users can feel a sense of unity and can increase their motivation for exercise.

In the above embodiment, synchronized video data is output to display units provided in a plurality of individual areas, but the video data may be output to a single display unit.

In that case, the video output system comprises: a storage unit that stores a plurality of video data items serving as exercise models; and a control unit that causes video data at an exercise level selected for each user from among the plurality of video data items and a captured image of the user to be output to a display unit provided for each user, wherein attribute information regarding a type of exercise is associated with each of the video data items, and the control unit determines a timing at which the start of exercise is prompted based on a start input from a user and the attribute information. According to this, exercise can be started from an exercise menu having an appropriate attribute, and the exercise effect can be further enhanced.

In determining the exercise menu, in addition to pain information, the exercise menu may be determined based on posture information obtained by evaluating the posture of each user. The posture information can be set for each body part such as the neck, shoulders, lower back, hip joints, knees, and the like, in the same manner as the pain information described above. The posture information may be input by a staff member from a tablet terminal or the like after examining the user's posture and range of motion of joints, may be input by the user himself or herself, or may be automatically evaluated by the control unit based on image information of the user acquired by the camera 30 or the like. Specifically, the posture and range of motion of each body part are evaluated in a plurality of stages, and a high evaluation is given when the posture is good or the range of motion is large, whereas a low evaluation is given when the posture is poor or the range of motion is small. In the present system, the control unit can increase the difficulty of the menu when the evaluation value of the posture information is equal to or greater than a predetermined value (high), and can decrease the difficulty of the menu when the evaluation value is equal to or less than a predetermined value (low).

In determining the exercise menu, the exercise menu may also be determined based on menu achievement degree information. For example, after a user actually performs a specific exercise menu, if the user has sufficient capacity after performing the specific exercise menu, the achievement degree may be evaluated as high, and in that case the difficulty of the menu may be increased; if the achievement degree is low, the difficulty of the menu may be decreased. The achievement degree information may be input by a staff member from a tablet terminal or the like after checking the user's exercise state, may be input by the user himself or herself, or may be automatically evaluated by the control unit based on image information of the user acquired by the camera 30 or the like.

The exercise menu may be reviewed (updated) at predetermined intervals, such as weekly, monthly, semiannually, or the like. The exercise menu is preferably reviewed by the control unit selecting an appropriate exercise menu based on various types of user information such as pain information, posture information, achievement degree information, and the like updated at each predetermined interval. A staff member may select the exercise menu based on the various types of user information. By appropriately reviewing the exercise menu at predetermined intervals in this way, the user can always perform exercise at a level appropriate for himself or herself.

Although a preferred embodiment of the present disclosure has been described in detail above with reference to the accompanying drawings, the technical scope of the present disclosure is not limited to such an example. It is obvious that a person having ordinary knowledge in the technical field of the present disclosure can conceive of various modifications or corrections within the scope of the technical idea described in the claims, and these are naturally understood to belong to the technical scope of the present disclosure.

The apparatus described in the present specification may be realized as a single apparatus, or part or all of the apparatus may be realized by a plurality of apparatuses (for example, cloud servers) connected by a network. For example, the control unit 11 and the storage unit 12 of the information processing apparatus 10 may be realized by different apparatuses connected to each other by a network.

A series of processing by the apparatus described in the present specification may be realized using any of software, hardware, and a combination of software and hardware. A computer program for realizing each function of the information processing apparatus according to the present embodiment can be created and implemented in a PC or the like. A computer-readable recording medium storing such a computer program can also be provided. The recording medium is, for example, a magnetic disk, an optical disk, a magneto-optical disk, a flash memory, or the like. The computer program described above may be distributed via a network, for example, without using a recording medium.

The processes described in the present specification using a flowchart do not necessarily have to be executed in the illustrated order. Several processing steps may be executed in parallel. Additional processing steps may be employed, and some processing steps may be omitted.

The effects described in the present specification are merely illustrative or exemplary and are not limiting. In other words, the technology according to the present disclosure may exhibit other effects that are apparent to those skilled in the art from the description of the present specification, together with or instead of the above effects.

The following configurations also belong to the technical scope of the present disclosure.

### (Item 1)

A video output system comprising:
a storage unit that stores a plurality of video data items serving as exercise models; and
a control unit that causes video data at an exercise level selected for each user from among the plurality of video data items and a captured image of the user to be output to a display unit provided for each user,
wherein the control unit outputs, to display units arranged in a plurality of individual areas respectively used by users, the video data synchronized with each other based on predetermined order information.

### (Item 2)

The video output system according to item 1, wherein the control unit repeatedly outputs the plurality of video data items to the display unit at a constant cycle based on the order information.

### (Item 3)

The video output system according to item 1 or 2, wherein the control unit selects the exercise level based on information regarding a painful body part of each user.

### (Item 4)

The video output system according to item 1 or 2, wherein the control unit selects the exercise level based on information regarding an age of each user.

### (Item 5)

The video output system according to item 1 or 2, wherein the control unit selects the exercise level based on information regarding a sex of each user.

### (Item 6)

The video output system according to item 1 or 2, wherein:
the storage unit stores history information regarding a history of each user using the video output system; and
the control unit selects the exercise level based on the history information of each user.

### (Item 7)

The video output system according to item 1 or 2, wherein the control unit outputs, to the display unit, image data prompting a user to start exercise based on a start input from the user.

### (Item 8)

The video output system according to item 7, wherein the control unit outputs, to the display unit, image data prompting an end of exercise after a predetermined period from output of the image data prompting the start of exercise.

### (Item 9)

The video output system according to item 7, wherein:
attribute information regarding a type of exercise is associated with each of the video data items; and
the control unit determines a timing at which the start of exercise is prompted based on the start input and the attribute information.

### (Item 10)

A video output method in which an information processing apparatus executes:
a storage process of storing a plurality of video data items serving as exercise models; and
a control process of causing video data at an exercise level selected for each user from among the plurality of video data items and a captured image of the user to be output to a display unit provided for each user,
wherein the control process includes a process of outputting, to display units arranged in a plurality of individual areas respectively used by users, the video data synchronized with each other based on predetermined order information.

### (Item 11)

A program causing an information processing apparatus to execute:
a storage process of storing a plurality of video data items serving as exercise models; and
a control process of causing video data at an exercise level selected for each user from among the plurality of video data items and a captured image of the user to be output to a display unit provided for each user,
wherein the control process includes a process of outputting, to display units arranged in a plurality of individual areas respectively used by users, the video data synchronized with each other based on predetermined order information.

### [Description of Reference Signs]

- 1: Video output system
- 10: Information processing apparatus
- 11: Control unit
- 12: Storage unit
- 13: Input unit
- 14: Communication unit
- 20: Display unit
- 30: Camera

## Claims

1. A video output system comprising:
a storage unit that stores a plurality of video data items serving as exercise models; and
a control unit that causes video data at an exercise level selected for each user from among the plurality of video data items and a captured image of the user to be output to a display unit provided for each user,
wherein the control unit outputs, to display units arranged in a plurality of individual areas respectively used by users, the video data synchronized with each other based on predetermined order information.

2. The video output system according to claim 1, wherein the control unit repeatedly outputs the plurality of video data items to the display unit at a constant cycle based on the order information.

3. The video output system according to claim 1 or 2, wherein the control unit selects the exercise level based on information regarding a painful body part of each user.

4. The video output system according to claim 1 or 2, wherein the control unit selects the exercise level based on information regarding an age of each user.

5. The video output system according to claim 1 or 2, wherein the control unit selects the exercise level based on information regarding a sex of each user.

6. The video output system according to claim 1 or 2, wherein:
the storage unit stores history information regarding a history of each user using the video output system; and
the control unit selects the exercise level based on the history information of each user.

7. The video output system according to claim 1 or 2, wherein the control unit outputs, to the display unit, image data prompting a user to start exercise based on a start input from the user.

8. The video output system according to claim 7, wherein the control unit outputs, to the display unit, image data prompting an end of exercise after a predetermined period from output of the image data prompting the start of exercise.

9. The video output system according to claim 7, wherein:
attribute information regarding a type of exercise is associated with each of the video data items; and
the control unit determines a timing at which the start of exercise is prompted based on the start input and the attribute information.

10. A video output method in which an information processing apparatus executes:
a storage process of storing a plurality of video data items serving as exercise models; and
a control process of causing video data at an exercise level selected for each user from among the plurality of video data items and a captured image of the user to be output to a display unit provided for each user, wherein the control process includes a process of outputting, to display units arranged in a plurality of individual areas respectively used by users, the video data synchronized with each other based on predetermined order information.

11. A program causing an information processing apparatus to execute:
a storage process of storing a plurality of video data items serving as exercise models; and
a control process of causing video data at an exercise level selected for each user from among the plurality of video data items and a captured image of the user to be output to a display unit provided for each user,
wherein the control process includes a process of outputting, to display units arranged in a plurality of individual areas respectively used by users, the video data synchronized with each other based on predetermined order information.
